# EUROPÄISCHE PATENTSCHRIFT

(11) **EP 3 104 928 B1**
(45) Veröffentlichungstag und Bekanntmachung des Hinweises auf die Patenterteilung: **18.10.2017**
(21) Anmeldenummer: 15706177.1
(22) Anmeldetag: 11.02.2015
(51) Int. Cl.: A61M 39/22

(54) **VORRICHTUNG ZUM STERILEN VERBINDEN VON MEDIZINISCHEN EINWEGARTIKELN**
DEVICE FOR CONNECTING MEDICAL DISPOSABLE ARTICLES IN A STERILE MANNER
DISPOSITIF DE RACCORDEMENT STÉRILE D'ARTICLES MÉDICAUX À USAGE UNIQUE

(30) Priorität: 11.02.2014 DE 202014100585 U
(43) Veröffentlichungstag der Anmeldung: 21.12.2016
(73) Patentinhaber: Heinz Meise GmbH, 58579 Schalksmühle (DE)
(72) Erfinder: MEISE, Heinz, 58579 Schalksmühle (DE)
(74) Vertreter: Patentanwälte Dörner & Kötter PartG mbB
(86) Internationale Anmeldenummer: PCT/EP2015/052855
(87) Internationale Veröffentlichungsnummer: WO 2015/121296

(56) Entgegenhaltungen:
- WO-A1-2006/122406
- WO-A1-2008/070220
- WO-A1-2008/094707
- US-A- 5 492 147
- US-A1- 2003 060 804
- US-A1- 2008 004 600
- US-A1- 2008 264 450
- US-A1- 2011 074 148
- US-A1- 2011 240 158

## Beschreibung

Die Erfindung betrifft eine Vorrichtung zum sterilen Verbinden von medizinischen Einwegartikeln, bestehend aus einem Auslaufkonnektor, der ein Rohrstück aufweist, das mit einer Dichtung und dazu benachbart mit Austrittsfenstern versehen ist und an seinem einen Ende mit einem Verschluss verschlossen ist, und einem Einlaufkonnektor, der eine Aufnahme aufweist, in der ein Führungselement angeordnet ist, wobei der Auslaufkonnektor und der Einlaufkonnektor an ihren einander zugewandten Enden jeweils mit einer Perforationsdichtung verschlossen sind.

Vorrichtungen der hier betrachteten Art finden in vielfältiger Weise im medizinischen Bereich Anwendung, beispielsweise im Bereich der Blutspenden, Bluttransfusionen, der Dialyse und dergleichen zum Verbinden von Schläuchen, Zentrifugenglocken usw. und sind beispielsweise aus der WO 2008/094707 A1 bekannt. Neben einer zuverlässigen Verbindung der jeweiligen Einwegartikel besteht ein wesentliches Kriterium solcher Vorrichtungen in der Sterilheit der Verbindung. Die Vorrichtung muss in jedem Falle gewährleisten, dass beim Verbinden der Einwegartikel keine Bakterien oder Fremdstoffe in die Verbindung gelangen, die dann möglicherweise mit der Flüssigkeit in den Körper des jeweiligen Patienten gelangen und dort Infektionen auslösen können.

Zur Bereitstellung einer sterilen Verbindung findet üblicherweise jeweils ein Sterilisationsvorgang der zu verbindenden Vorrichtungen statt. Dies kann auf verschiedene Weise erfolgen, beispielsweise mittels Heißdampf in einem Autoklaven. Auch die Verwendung von steril verpackten Enden, die erst unmittelbar vor der Benutzung ausgepackt und direkt verbunden werden, ist bekannt. Die bekannten Lösungen sind jedoch zum einen aufwendig, zum anderen gewährleisten sie keine vollständig sterile Verbindung.

Zur Vermeidung des Sterilisiervorgangs für die zu verbindenden Teile ist aus der DE 20 2009 008 274 U1 eine Vorrichtung zum sterilen Verbinden von medizinischen Einwegartikeln bekannt, bei der das Rohrstück mit seinen Austrittsfenstern in dem Führungselement verfahrbar angeordnet ist. In dem Führungselement sind Durchtrittsöffnungen ausgebildet. Weiterhin ist auf dem Rohrstück eine Hülse verschiebbar angeordnet, auf der eine Überwurfmutter vorgesehen ist. Die bekannte Vorrichtung erfüllt die an sie gestellten Anforderungen hinsichtlich der Sterilität der Verbindung. Allerdings hat sich gezeigt, dass es beim Durchfluss beispielsweise von Blut durch die bekannte Vorrichtung zu einer leichten Schädigung des Blutes kommen kann. So wurde zum Beispiel eine Schädigung der äußeren Haut der roten Blutkörperchen beobachtet, was beim Austritt des Blutes aus den Durchtrittsöffnungen auftritt. Durch diese Beschädigungen kann Hämoglobin freigesetzt werden, was die Qualität des Blutes reduziert.

Hier will die Erfindung Abhilfe schaffen. Der Erfindung liegt die Aufgabe zugrunde, eine Vorrichtung zum sterilen Verbinden von medizinischen Einwegartikeln zu schaffen, die einerseits die geforderte Sterilität beim Verbinden ermöglicht und andererseits eine Beschädigung der durch die Vorrichtung strömenden Medien vermeidet. Gemäß der Erfindung wird diese Aufgabe durch die Merkmale des Patentanspruchs 1 gelöst.

Mit der Erfindung ist eine Vorrichtung zum sterilen Verbinden von medizinischen Einwegartikeln geschaffen, die einerseits die geforderte Sterilität beim Verbinden ermöglicht und andererseits eine Beschädigung der durch die Vorrichtung strömenden Medien vermeidet. Gleichzeitig ist die Handhabung der Verbindung einfach.

Andere Weiterbildungen und Ausgestaltungen der Erfindung sind in den übrigen Unteransprüchen angegeben. Ausführungsbeispiele der Erfindung sind in der Zeichnung dargestellt und werden nachfolgend im Einzelnen beschrieben. Es zeigen:
- Fig. 1: die Ansicht eines Ein- und eines Auslaufkonnektors mit Schutzkappen;
- Fig. 2: den schnitt entlang der Linie D-D in Figur 1;
- Fig. 3: die Ansicht der Vorrichtung in miteinander verbundenem Zustand von Einlauf- und Auslaufkonnektoren;
- Fig. 4: die Seitenansicht von rechts auf die in Figur 3 dargestellte Vorrichtung;
- Fig. 5: die Seitenansicht von links auf die in Figur 3 dargestellte Vorrichtung
- Fig. 6: den Schnitt entlang der Linie C-C in Figur 3;

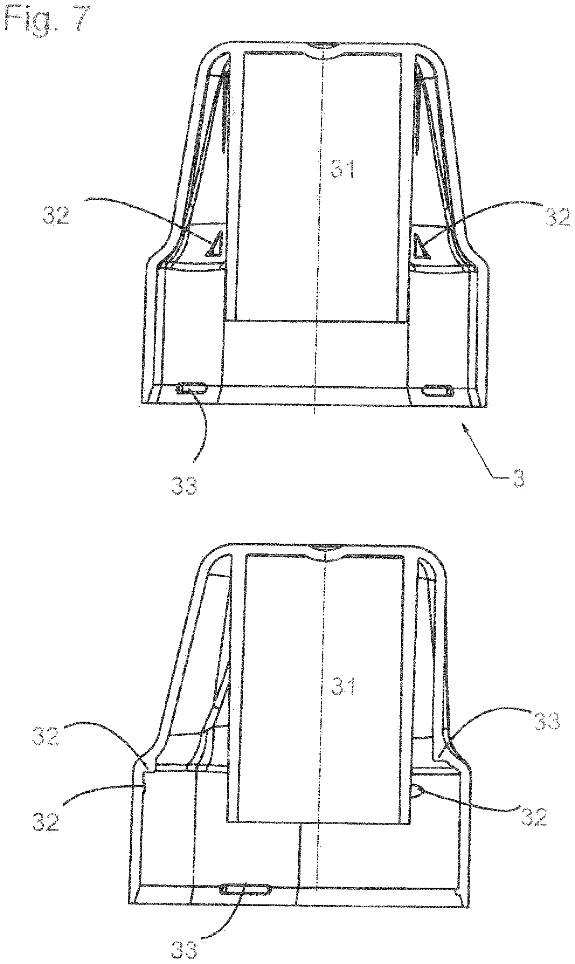

- Fig. 7: den Schnitt durch eine Schutzkappe;

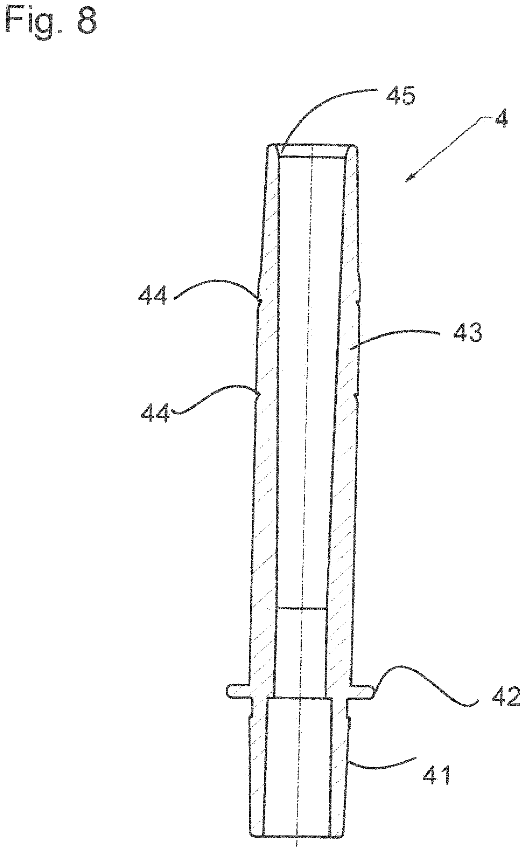

- Fig. 8: den Schnitt durch einen Auslaufzylinder;;

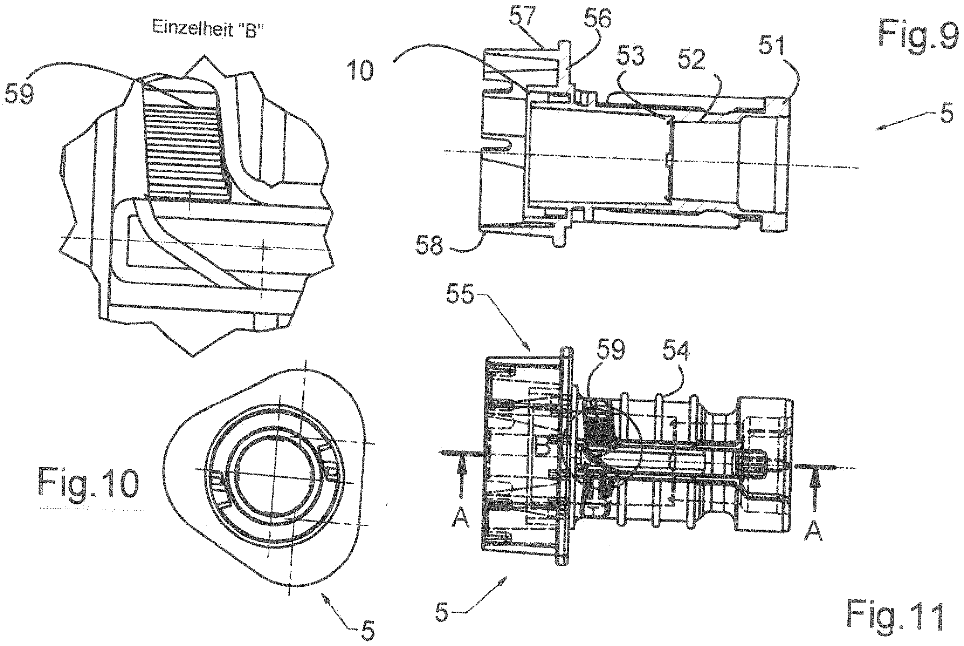

- Fig. 9: den Schnitt durch ein Auslauftorso;
- Fig. 10: die Seitenansicht von links des in Figur 9 dargestellten Auslauftorsos;
- Fig. 11: die Ansicht des in Figur 9 dargestellten Auslauftorsos;

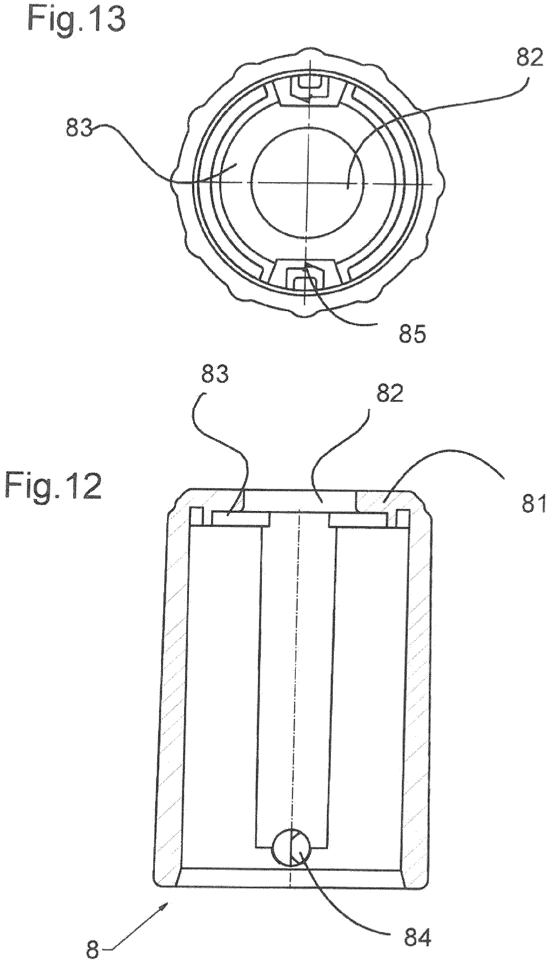

- Fig. 12: den Schnitt durch eine Überwurfmutter;
- Fig. 13: die Seitenansicht von links der in Figur 12 dargestellten Überwurfmutter;

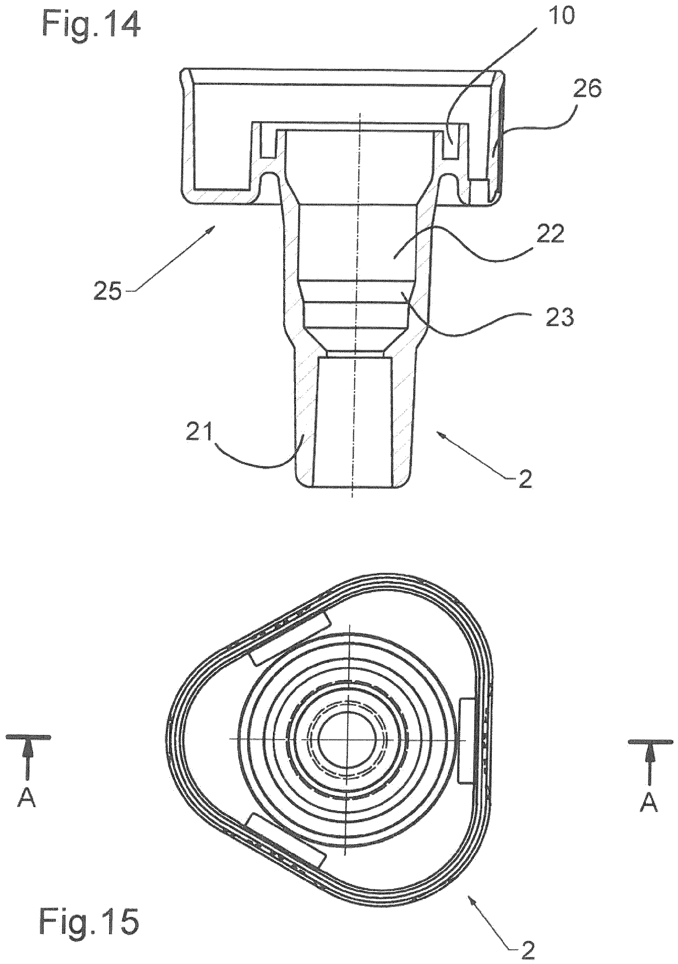

- Fig. 14: den Schnitt durch einen Einlaufkonnektor;
- Fig. 15: die Seitenansicht von rechts des in Figur 14 dargestellten Einlaufkonnektors;

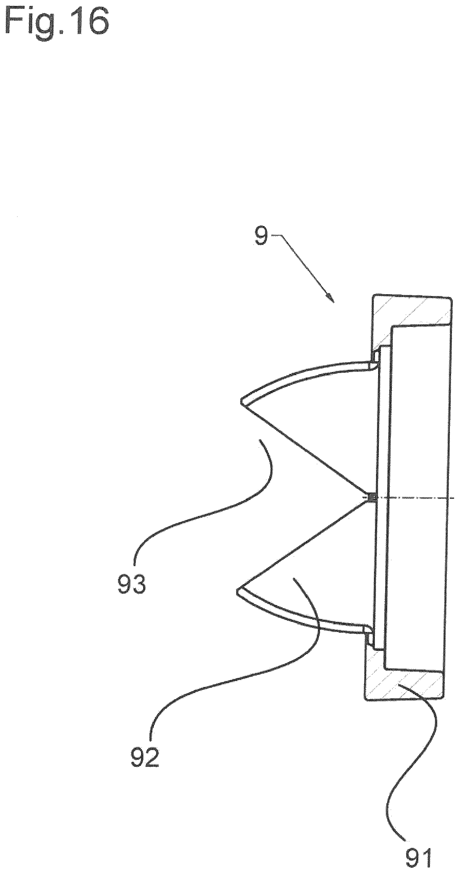

- Fig. 16: den Schnitt durch eine Perforationsdichtung;

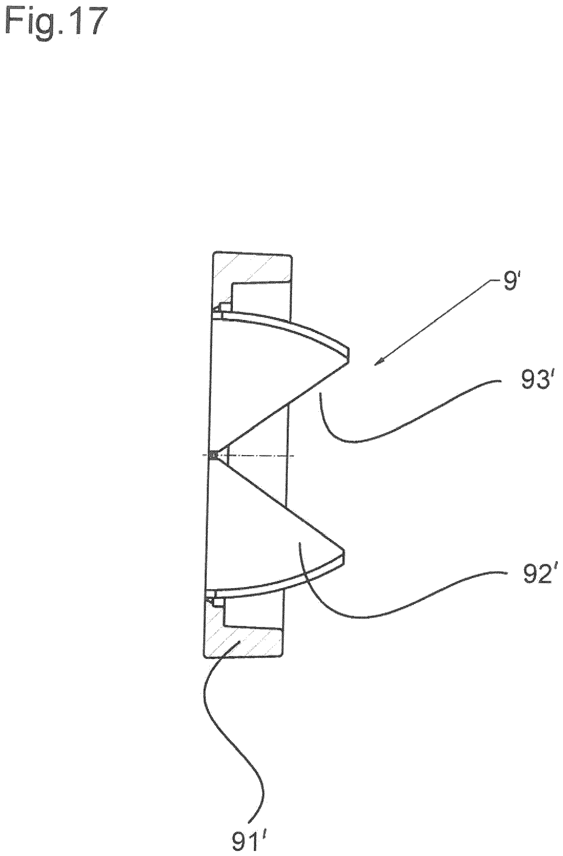

- Fig. 17: den Schnitt durch eine Perforationsdichtung in anderer Ausgestaltung;

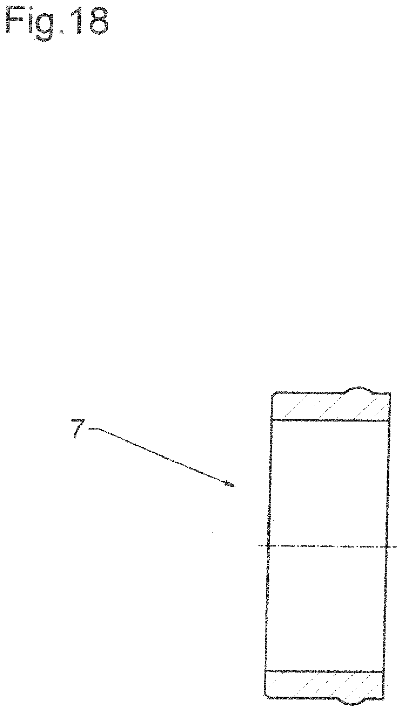

- Fig. 18: den Schnitt durch einen Schnappring;
- Fig. 19: den Schnitt durch einen Einlaufkonnektor mit Abbrechkonnektor und Abbrechteil;
- Fig. 20: den Schnitt durch einen Einlaufkonnektor mit Abbrechteil ohne Abbrechkonnektor;
- Fig. 21: den Schnitt durch einen Einlaufkonnektor mit Abbrechkonnektor und Abbrechteil in anderer Ausgestaltung;
- Fig. 22: den Schnitt durch den in Figur 21 dargestellten Einlaufkonnektor;
- Fig. 23: den Schnitt durch einen Abbrechteil mit einer Hülse.

Die als Ausführungsbeispiel gewählte Vorrichtung zum sterilen Verbinden von medizinischen Einwegartikeln besteht aus einem Auslaufkonnektor 1 und einem Einlaufkonnektor 2, die verschiebbar ineinander greifen. Auslaufkonnektor 1 und Einlaufkonnektor 2 sind vor ihrer Verbindung jeweils mit einer Verschlusskappe 3 verschossen.

Der Auslaufkonnektor 1 weist einen Auslaufzylinder 4 auf, der rotationssymmetrisch ausgebildet ist. Der Auslaufzylinder 4 ist an seinem einen, dem Einlaufkonnektor 2 abgewandten Ende mit einem konisch ausgebildeten Schlauchsitz 41 versehen, der zu Verbindung mit dem jeweiligen Schlauch dient. An den Schlauchsitz 41 schließt sich ein Anschlag 42 an, der tellerartig ausgebildet ist und an den sich ein zylindrischer Abschnitt 43 anschließt. Auf dem Abschnitt 43 sind Raststellen 44 vorgesehen, in deren Anschluss der Auslaufzylinder 4 konisch zu seinem freien Ende hin zuläuft. Das Ende ist von einer Dichtlippe 45 gebildet.

Der Auslaufkonnektor 1 umfasst darüber hinaus einen Auslauftorso 5, in dem der Auslaufzylinder 4 verschiebbar angeordnet ist. Sein eines Ende ist von einem Ring 51 gebildet, an den sich ein Abschnitt 52 geringeren Durchmessers anschließt. Der Innendurchmesser des Abschnitts 52 korrespondiert mit dem Außendurchmesser des Abschnitts 43 des Auslaufzylinders 4. Der Abschnitt 52 ist innen begrenzt durch eine Rastnase 53, die in montiertem Zustand mit den Raststellen 44 des Auslaufzylinders 4 zusammenwirkt. Außen ist der Auslaufzylinder 4 mit im Ausführungsbeispiel drei Rippen 54 versehen.

Das dem Ring 51 abgewandte Ende des Auslauftorsos 5 ist von einem Kupplungsteil 55 gebildet. Das Kupplungsteil 55 besteht aus einem Flansch 56, an den ein Überstand 57 angeformt ist, der parallel zur Längsmittellinie des Auslauftorsos 5 ausgerichtet ist und der das stirnseitige Ende des Auslauftorsos 5 überragt. Der Überstand 57 hat eine im Wesentlichen dreieckige Kontur. Das freie Ende des Überstands 57 weist eine Rastnase 58 auf. Zwischen den Rippen 54 und dem Flansch 56 ist eine Verriegelungseinrichtung 59 angeordnet, die sägezahnartig ausgebildet ist. In das stirnseitige Ende des Auslauftorsos 5 ist eine Ringnut 10 eingebracht.

In montiertem Zustand ist zwischen Auslaufzylinder 4 und Auslauftorso 5 eine Dichtung 6 in Form eines O-Rings angeordnet. Die Dichtung 6 liegt einerseits auf dem zylindrischen Abschnitt 43 des Auslaufzylinders 4 auf, andererseits liegt die Dichtung 6 am Fuß des Rings 51 des Auslaufzylinders 5 an. Die Dichtung 6 ist durch einen Schnappring 7 gesichert.

An dem Auslaufkonnektor 1 ist eine Überwurfmutter 8 vorgesehen, die rotationssymmetrisch ausgebildet ist. Sie ist im Ausführungsbeispiel einstückig hergestellt. Die Überwurfmutter 8 ist außen mit einer Riffelung versehen, die zu einer Verbesserung bei der Handhabung führt. An ihrem einen Ende ist die Überwurfmutter 8 mit einem Deckel 81 verschlossen, der in seiner Mitte eine Öffnung 82 aufweist, die in montiertem Zustand zum Durchtritt des Auslaufzylinders 4 dient. Um die Öffnung 82 ist innen eine Vertiefung 83 ausgebildet, in der sich in montiertem Zustand der Anschlag 42 des Auslaufzylinders 4 abstützt. Auf der dem Deckel 81 abgewandten Ende ist die Überwurfmutter 8 offen ausgestaltet. In dem endseitigen Bereich ist sie mit einer Verzahnung 84 versehen, die mit der Verriegelungseinrichtung 59 des Auslauftorsos 5 korrespondiert. Benachbart zur der Verzahnung 84 sind innen in der Überwurfmutter 8 zwei sich diametral gegenüberliegende Führungsnoppen 85 angeordnet, die in der Endposition der Überwurfmutter 8 in der Verriegelungseinrichtung 59 des Auslauftorsos 5 enrasten.

Der Auslaufkonnektor 1 ist an seinem dem Einlaufkonnektor 2 zugewandten Ende mit einer Perforationsdichtung 9 verschlossen, die aus einem Elastomer oder Silikon, besteht. Sie weist einen ringartigen Grundkörper 91 auf, der in montiertem Zustand in der Ringnut 10 im Auslaufzylinders 5 positioniert ist. An den Grundkörper 91 ist ein Perforationselement 92 angeformt. Das Perforationselement 92 kann in Abwandlung des Ausführungsbeispiels auch im 2-Komponenten-Spritzgussverfahren in den Grundkörper 91 eingebracht sein.

Der Einlaufkonnektor 2 ist an seinem einen, dem Auslaufkonnektor 1 abgewandten Ende mit einem konisch ausgebildeten Schlauchsitz 21 versehen, der zu Verbindung mit dem jeweiligen Schlauch dient. Der Einlaufkonnektor 2 ist von einer Bohrung 22 durchsetzt, die in ihrem mittleren Bereich mehrfach abgesetzt ist. Dadurch ist ein Dichtkonus 23 hervorgerufen, an dem in geöffnetem Zustand der Vorrichtung die Dichtlippe 45 des Auslaufzylinders 4 anliegt.

An seinem dem Schlauchsitz 21 abgewandten Ende ist an dem stirnseitigen Ende ein Kupplungsteil 25 angeordnet. Das Kupplungsteil 25 besteht aus einem Überstand 26, der parallel zur Längsmittellinie des Einlaufkonnektors 2 ausgerichtet ist und der das stirnseitige Ende des Einlaufkonnektors 2 überragt. Der Überstand 26 hat eine im Wesentlichen dreieckige Kontur. Die lichte Weite des Überstands 26 entspricht im Wesentlichen den äußeren Abmessungen des Überstands 57 des Kupplungsteils 55, so dass der Überstand 57 bei der Montage der Vorrichtung in den Überstand 26 einfährt. In das stirnseitige Ende des Einlaufkonnektors 2 ist eine Ringnut 10' eingebracht.

Der Einlaufkonnektor 2 ist an seinem dem Auslaufkonnektor 1 zugewandten Ende mit einer Perforationsdichtung 9' verschlossen, die ebenfalls aus einem Elastomer oder Silikon, besteht. Sie weist einen ringartigen Grundkörper 91' auf, der in montiertem Zustand in der Ringnut 10' im Einlaufkonnektor 2 positioniert ist. An den Grundkörper 91' ist ein Perforationselement 92' angeformt, welches sich in die Bohrung 22 des Einlaufkonnektors 2 erstreckt.

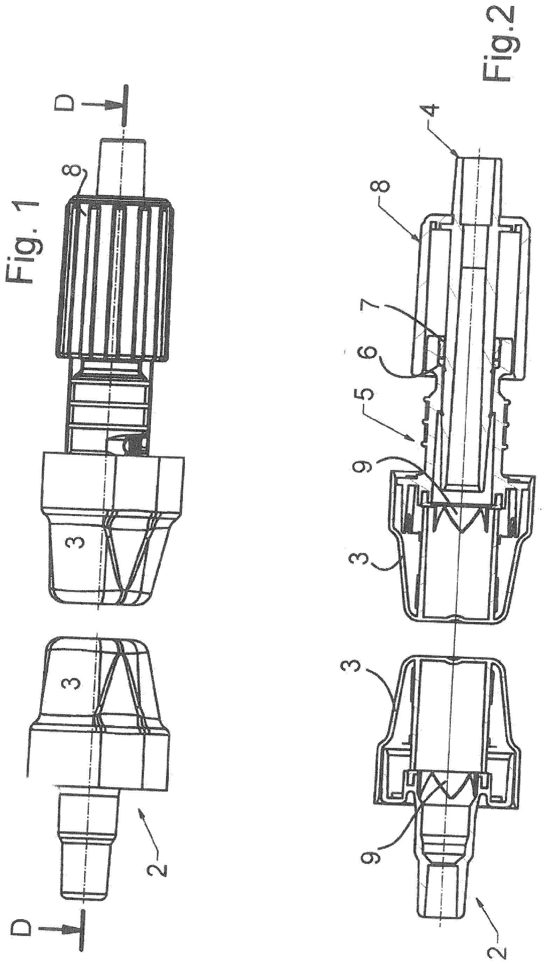

In nicht verbundenem Zustand der Vorrichtung (Figur 2) sind der Auslaufkonnektor 1 und der Einlaufkonnektor 2 jeweils mit der Verschlusskappe 3 verschlossen. Die Verschlusskappe 3 hat in der Ansicht eine im Wesentlichen dreieckförmige Kontur, die mit der Form der Überstände 26 und 57 korrespondiert. Die Schutzkappe 3 ist innen mit einem Führungszylinder 31 versehen, dessen Durchmesser dem Durchmesser der stirnseitigen, einander zugewandten Enden des Einlaufkonnektors 2 sowie des Auslauftorsos 5 entspricht. Außen sind auf dem Führungszylinder 31 über seinen Umfang verteilt Vorsprünge 32 angeordnet. Im Bereich seines offenen Endes weist die Schutzkappe 3 drei Rastbögen 33 auf.

In verschlossenem Zustand stützt sich der Führungszylinder 31 unmittelbar benachbart zu den Ringnuten 24 und 56 auf den stirnseitigen Enden des Einlaufkonnektors 2 sowie des Auslauftorsos 5 ab, ohne dabei mit den Perforationsdichtungen 9, 9' in Kontakt zu treten. Gleichzeitig liegen die Überstände 26 und 57 an den Vorsprüngen 32 an. Die Rastbögen 33 fassen in diesem Zustand hinter das Kupplungsteil 25 bzw. den Flansch 56, wodurch eine zuverlässige Anordnung gewährleistet ist.

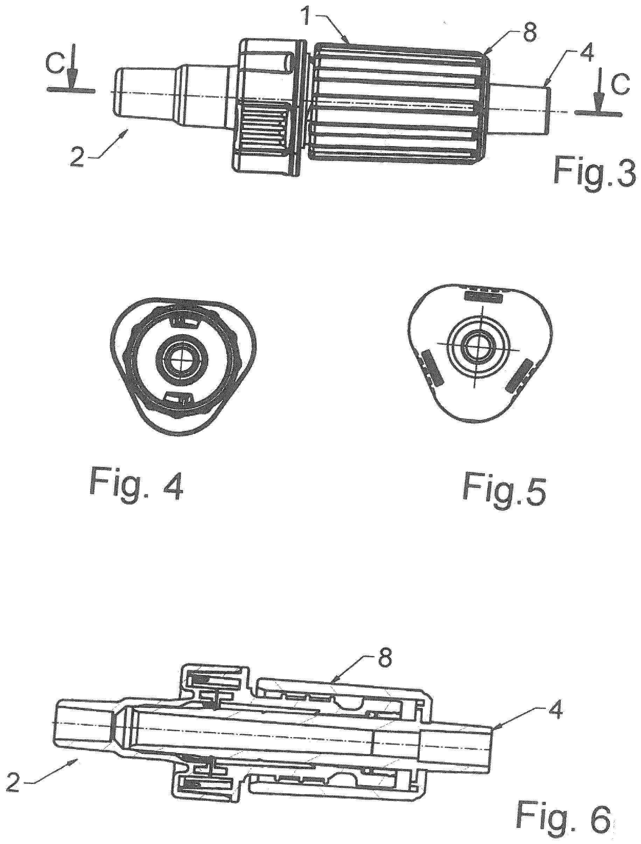

Zur Verbindung von Auslaufkonnektor 1 und Einlaufkonnektor 2 werden zunächst die Verschlusskappen 3 entfernt. Sodann werden der Auslaufkonnektor 1 und der Einlaufkonnektor 2 aufeinander zugeführt. Dabei fahren der der Überstand 26 und der Überstand 57 ineinander. In ihrer Endposition verrasten die Konnektoren 1 und 2 mit Hilfe der Rastnasen 53 am Auslauftorso 5. Erkennbar stoßen in dieser Position die stirnseitigen Enden des Auslaufkonnektors 1 und des Einlaufkonnektors 2 spaltfrei aneinander (Figur 6). Gleichzeitig liegen die Perforationsdichtungen 9 und 9' deckungsgleich übereinander. Die Konnektoren 1 und 2 sind in diesem Zustand noch gegeneinander abgedichtet, so dass noch kein Medium von einem in den anderen Konnektor gelangen kann.

Zur Freigabe des Durchflusses wird die Überwurfmutter 8 in Richtung des Einlaufkonnektors 2 bewegt. Aufgrund des sich in der Vertiefung 83 der Überwurfmutter 8 abstützenden Anschlags 42 des Auslaufzylinders 4 führt diese Verschiebung der Überwurfmutter 8 gleichzeitig zu einer axialen Bewegung des Auslaufzylinders 4 in Richtung des Einlaufkonnektors 2. Diese Bewegung wird ausgeführt, bis die Verzahnung 84 der Überwurfmutter 8 mit der Verriegelungseinrichtung 59 in Kontakt tritt. Da diese eine wendelartige Kontur aufweist, führt eine Drehung der Überwurfmutter 8 gleichzeitig zu einer weiteren axialen Bewegung der Überwurfmutter 8 sowie des Auslaufzylinders 4 in Richtung des Einlaufkonnektors 2. Bei ihrer axialen Bewegung durchstößt die Dichtlippe 45 die Perforationsdichtungen 9 und 9', wodurch die Perforationsdichtungen 9, 9' aufreißen und jeweils eine V-förmige Einkerbung 93, 93' entsteht. Ein Durchfließen der Vorrichtung ist dann ermöglicht. Die Endposition ist erreicht, wenn die Überwurfmutter 8 an dem Flansch 56 anliegt. Ebenso liegt dann die Dichtlippe 45 an dem Dichtkonus 23 des Einlaufkonnektors 2 an. Der Durchfluss des jeweiligen Mediums ist somit möglich, wobei die Konnektoren 1 und 2 zuverlässig gegeneinander abgedichtet sind. Ein versehentliches Lösen der Konnektoren 1 und 2 ist darüber hinaus aufgrund der sägezahnartigen Ausbildung der Verzahnung 84 verhindert, die ein Zurückdrehen der Überwurfmutter 8 und damit ein Öffnen der Verbindung wirksam verhindert.

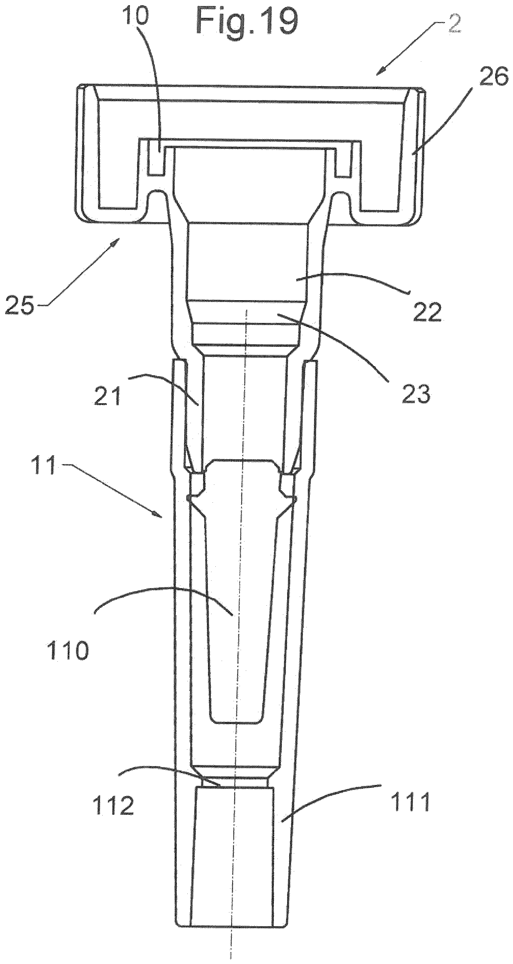

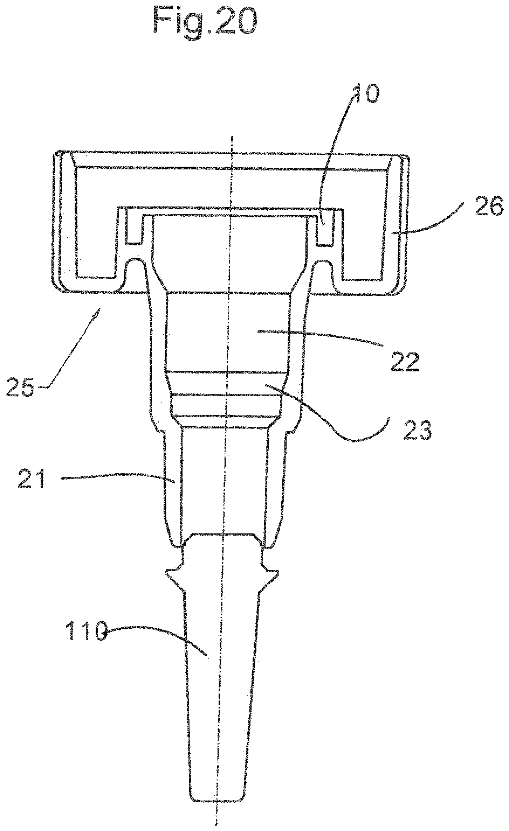

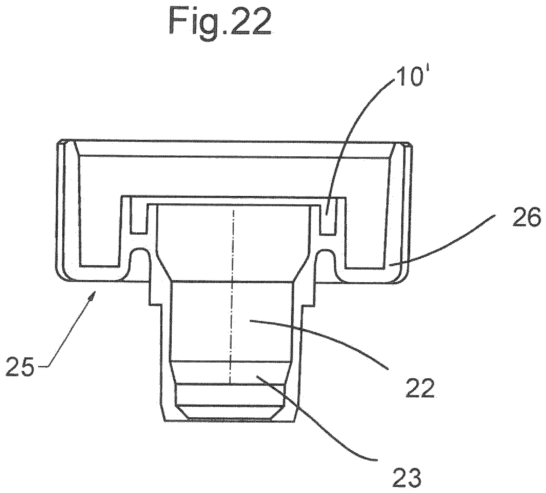

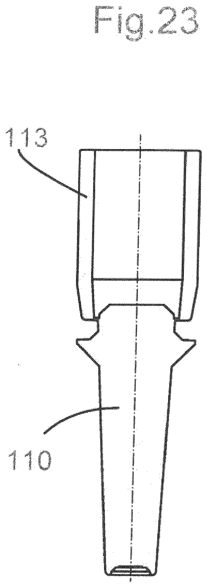

In Ergänzung der vorstehend beschriebenen Vorrichtung besteht die Möglichkeit, am Einlaufkonnektor 2 einen Abbrechkonnektor 11 vorzusehen, wie er im Ausführungsbeispiel nach den Figuren 19 bis 23 dargestellt ist. Dabei findet in der Ausführung nach den Figuren 19 und 20 prinzipiell der Einlaufkonnektor 2 in seiner oben beschriebenen Form Anwendung. Zusätzlich ist jedoch an den Schlauchsitz 21 ein Abbrechteil 110 in Form eines Stößels angeformt, welches mit dem stirnseitigen Ende des Schlauchsitzes 21 über eine Art Filmscharnier verbunden ist (vgl. Figur 20). In diesem Zustand ist die Bohrung 22 durch das Abbrechteil 110 verschlossen. Auf den Schlauchsitz 21 ist zusätzlich ein Schlauchadapter 111 geschoben und verklebt, der aus flexiblem Material besteht. Der Schlauchadapter 111 umgibt das Abbrechteil 110 vollständig. Beabstandet zum freien Ende des Abbrechteils 110 ist in dem Schlauchadapter 111 eine Querschnittsverjüngung 112 ausgebildet.

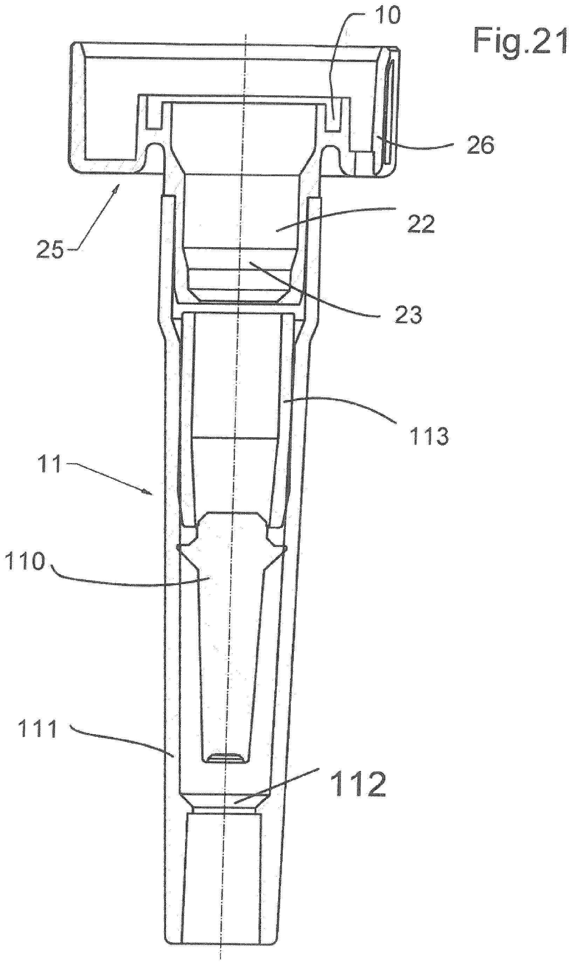

Im Ausführungsbeispiel nach den Figuren 21 bis 23 ist der Einlaufkonnektor 2 derart geändert, dass der Schlauchsitz 21 weggelassen ist. Vielmehr ist auf die die Bohrung 22 umgebende Wandung der Schlauchadapter 111 aufgeschoben und verklebt. Der Schlauchadapter 111 weist ebenfalls die Querschnittsverjüngung 112 auf. Der Abbrechkonnektor 11 umfasst auch in dieser Ausführung das Abbrechteil 110. Es ist jedoch nicht an den Einlaufkonnektor 2 angeformt, sondern an eine Hülse 113, die gemeinsam mit dem Abbrechteil 110 von dem Schlauchadapter 111 umgeben ist.

Die erfindungsgemäße Vorrichtung stellt eine sterile Verbindungsmöglichkeit von medizinischen Einwegartikeln bereit. Dies ist dadurch gewährleistet, dass in nicht verbundenem Zustand der jeweiligen medizinischen Einwegartikel einerseits der Auslaufkonnektor 1 in seinem dem Schlauch abgewandten Ende durch die Perforationsdichtung 9 verschlossen ist. In Folge dessen ist der für den Durchfluss der jeweiligen Flüssigkeit vorgesehene innere Bereich des Auslaufzylinders 4 gegen die Umgebung abgedichtet. Dies gilt auch nach Abziehen der Verschlusskappen 3. Durch die Schutzkappe 3 ist außerdem eine versehentliche Beschädigung der Dichtung 9 verhindert.

Ebenso erfolgt die Abdichtung auf Seiten des Einlaufkonnektors 2 mit Hilfe der Perforationsdichtung 9', der die für den Durchfluss der Flüssigkeit vorgesehene Bohrung 22 des Einlaufkonnektors 2 gegen die Umgebung abdichtet, und zwar auch nach Abziehen der Schutzkappe 3. Auch ist durch die Schutzkappe 3 eine versehentliche Beschädigung der Dichtung 9' verhindert.

Der Durchfluss durch die Vorrichtung wird erst durch das Verschieben des Auslaufzylinders 4 in Richtung Einlaufkonnektors 2 und das Eintreten der Dichtlippe 45 in den Konnektor 2 und Durchstoßen der Dichtungen 9 und 9' freigegeben. Da in diesem Zustand jedoch die Kupplungsteile 25 und 55 bereits ineinander gefahren sind, ist auch insoweit ein Kontakt mit der Umgebung ausgeschlossen. Folglich ist durch die Positionierung der Dichtungen 9, 9' in der Bohrung 22 des Konnektors 2 eine sterile Verbindung hervorgerufen. Aufgrund der zurück versetzten Anordnung der Dichtungen 9 und 9' ist eine versehentliche Kontamination nach Abziehen der Schutzkappen 3 verhindert. Durch die verschiedenen Rastverbindungen ist darüber hinaus eine einfache Montage sowie eine hohe Stabilität der Verbindung hervorgerufen.

In den Ausführungsbeispielen nach den Figuren 19 bis 23 wird der Durchfluss darüber hinaus erst freigegeben, wenn der Abbrechkonnektor 11 betätigt wird. Dies erfolgt durch einfaches Kneten oder Biegen des Schlauchadapters 111 in dem dem Einlaufkonnektor 2 zugewandten Bereich des Abbrechteils 110. Hierdurch wird die Verbindung zwischen Abbrechteil 110 und Schlauchsitz 21 beziehungsweise Hülse 113 gelöst, so dass der Durchfluss durch die Vorrichtung freigegeben wird.

Die erfindungsgemäße Vorrichtung stellt zudem einen Durchfluss sicher, bei dem das Medium die Vorrichtung beschädigungsfrei durchströmen kann. Dies ist eine Folge der Bereitstellung eines glatten, rohrförmigen Durchströmbereichs ohne scharfe Kanten. Sobald nämlich das Medium aus dem Schlauch in den Einlaufkonnektor 2 gelangt, tritt es hinter dem abgesetzten Abschnitt im Bereich des Dichtkonus 23 in den Auslaufzylinder 4 ein, der innen glatt ausgebildet ist und von dem aus das Medium in den an den Auslaufkonnektor 1 angeschlossenen Schlauch gelangt.

Weiterhin stellt die im Wesentlichen dreieckige Kontur der Kupplungsteile 25 und 55 eine fehlerfreie Bedienung der Vorrichtung sicher. Das gleiche gilt für die ebenfalls dreieckige Ausgestaltung der Schutzkappen 3. Insgesamt ist die Handhabung der Vorrichtung sehr einfach und Bedienfehler sind vermieden.

## Patentansprüche

1. Vorrichtung zum sterilen Verbinden von medizinischen Einwegartikeln, bestehend aus einem Auslaufkonnektor (1), der einen Auslaufzylinder (4) aufweist, der mit einer Dichtung (6) versehen ist und der an seinem einen Ende verschlossen ist, wobei an dem Auslaufkonnektor (1) eine Überwurfmutter (8) vorgesehen ist, und einem Einlaufkonnektor (2), der mit dem Auslaufkonnektor (1) verbindbar ist und der an seinem einen Ende verschlossen ist, wobei der Auslaufkonnektor (1) und der Einlaufkonnektor (2) an ihren einander zugewandten Enden jeweils mit einer Perforationsdichtung (9, 9') verschlossen sind, **dadurch gekennzeichnet, dass** der Auslaufkonnektor (1) einen Auslauftorso (5) mit einem Kupplungsteil (55) umfasst, das mit einem Kupplungsteil (25) des Einlaufkonnektors (2) zusammenwirkt, wobei die Kupplungsteile (25) und (55) eine im Wesentlichen dreieckige Kontur haben, die eine fehlerfreie Bedienung der Vorrichtung sicherstellt, und die Überwurfmutter (8) mit einer sägezahnartigen Verzahnung (84) versehen ist, die mit einer Verriegelungseinrichtung (59) des Auslauftorsos (5) korrespondiert, wobei innen in der Überwurfmutter (8) Führungsnoppen (85) angeordnet sind, die in der Endposition der Überwurfmutter (8) in der Verriegelungseinrichtung (59) des Auslauftorsos (5) einrasten, die ein Zurückdrehen der Überwurfmutter (8) verhindern, und die Verriegelungseinrichtung (59) eine wendelförmige Kontur aufweist.

2. Vorrichtung nach Anspruch 1, **dadurch gekennzeichnet, dass** die Perforationsdichtungen (9, 9') einen Grundkörper (91, 91') aufweisen der in montiertem Zustand in einer Ringnut (10, 10') im Einlaufkonnektor (2) und / oder Auslaufzylinder (5) positioniert ist.

3. Vorrichtung nach Anspruch 1 oder 2, **dadurch gekennzeichnet, dass** die Perforationsdichtungen (9, 9') aus einem Elastomer oder Silikon bestehen.

4. Vorrichtung nach einem oder mehreren der vorgenannten Ansprüche, **dadurch gekennzeichnet, dass** die Perforationsdichtungen (9, 9') ein Perforationselement (92, 92') aufweisen.

5. Vorrichtung nach einem oder mehreren der vorgenannten Ansprüche, **dadurch gekennzeichnet, dass** Auslaufkonnektor (1) und Einlaufkonnektor (2) in ihrer Endposition verrasten und die Perforationsdichtungen (9, 9') deckungsgleich übereinander liegen.

6. Vorrichtung nach einem oder mehreren der vorgenannten Ansprüche, **dadurch gekennzeichnet, dass** an dem Einlaufkonnektor (2) ein Abbrechkonnektor (11) vorgesehen ist.

## Claims

1. Device for connecting disposable medical articles in a sterile manner consisting of an outlet connector (1) which has an outlet cylinder (4) which is provided with a seal (6) and which is closed at one of its ends, where a union nut (8) is provided on the outlet connector (1), and an inlet connector (2) which can be connected to the outlet connector (1) and which is closed at one of its ends, where the outlet connector (1) and the inlet connector (2) are each closed with a perforation seal (9, 9') at the ends facing one another, **characterised in that** the outlet connector (1) comprises an outlet torso (5) with a coupling section (55) which interacts with a coupling section (25) of the inlet connector (2), where the coupling sections (25) and (55) have a substantially triangular contour which ensures error-free operation of the device, and where the union nut (8) is provided with a serrated denticulation (84) corresponding to a locking device (59) on the outlet torso (5), where guiding knobs (85) are arranged on the inside of the union nut (8) which, in the end position of the union nut (8), engage the locking device (59) of the outlet torso (5) and prevent the union nut (8) from rotating back, and where the locking device (59) has a spiral-shaped contour.

2. Device in accordance with claim 1, **characterised in that** the perforation seals (9, 9') have a main body (91, 91') which, in the assembled state, is positioned in a ring groove (10, 10') in the inlet connector (2) and/or the outlet cylinder (5).

3. Device in accordance with claim 1 or claim 2, **characterised in that** the perforation seals (9, 9') consist of an elastomer or silicon.

4. Device in accordance with any one or several of the preceding claims, **characterised in that** the perforation seals (9, 9') have a perforation element (92, 92').

5. Device in accordance with any one or several of the preceding claims, **characterised in that**, in their end position, the outlet connector (1) and the inlet connector (2) interlock and the perforation seals (9, 9') fit identically on top of one another.

6. Device in accordance with any one or several of the preceding claims, **characterised in that** a break-off connector (11) is provided on the inlet connector (2).

## Revendications

1. Dispositif de raccordement stérile d'articles médicaux à usage unique, comprenant un connecteur de sortie (1) qui présente un cylindre de sortie (4) qui est muni d'un joint (6) et qui est obturé à l'une de ses extrémités, sachant que contre le connecteur de sortie (1) est prévu un écrou raccord (8), et comprenant un connecteur d'entrée (2) reliable au connecteur de sortie (1) et qui est obturé à l'une de ses extrémités, sachant que le connecteur de sortie (1) et le connecteur d'entrée (2) sont obturés, au niveau de leurs extrémités se faisant face, à l'aide d'un joint de perforation (9, 9'), **caractérisé en ce que** le connecteur de sortie (1) comprend un corps de sortie (5) avec une pièce d'accouplement (55) qui interagit avec une pièce d'accouplement (25) du connecteur d'entrée (2), sachant que les pièces d'accouplement (25) et (55) présentent un contour essentiellement triangulaire assurant une utilisation sans erreur du dispositif et que l'écrou raccord (8) est muni d'une denture (84) en dents de scie épousant un dispositif de verrouillage (59) du corps de sortie (5), sachant qu'à l'intérieur sont disposés dans l'écrou raccord (8) des tétons de guidage (85) qui encrantent dans le dispositif de verrouillage (59) du corps de sortie (5) lorsque l'écrou raccord (8) se trouve en position finale, tétons qui empêchent l'écrou raccord (8) de se dévisser, et sachant que le dispositif de verrouillage (59) présente un contour spiralé.

2. Dispositif selon la revendication 1, **caractérisé en ce que** les joints de perforation (9, 9') présentent un corps de base (91, 91') qui à l'état monté est positionné dans une rainure annulaire (10, 10') dans le connecteur d'admission (2) et/ou le cylindre de sortie (5).

3. Dispositif selon la revendication 1 ou 2, **caractérisé en ce que** les joints de perforation (9, 9') sont composés d'un élastomère ou d'une silicone.

4. Dispositif selon l'une ou plusieurs des revendications précédentes, **caractérisé en ce que** les joints de perforation (9, 9') présentent un élément de perforation (92, 92').

5. Dispositif selon l'une ou plusieurs des revendications précédentes, **caractérisé en ce que** le connecteur de sortie (1) et le connecteur d'admission (2) encrantent dans leur position finale et que les joints de perforation (9, 9') se trouvent en superposition exacte.

6. Dispositif selon l'une ou plusieurs des revendications précédentes, **caractérisé en ce que** sur le connecteur d'admission (2) est prévu un connecteur de rupture (11).
